# EUROPEAN PATENT APPLICATION

(11) **EP 4 795 940 A1**
(43) Date of publication of application: **26.08.2026**
(21) Application number: 24878502.4
(22) Date of filing: 05.06.2024
(51) Int. Cl.: A23L 33/00, A23L 33/19, A23L 33/185, A23L 33/115, A23L 33/125, A23L 33/21, A23L 33/10, A23L 33/175, A23L 33/15, A23L 33/155, A23L 33/16

(54) **NUTRITIONAL COMPOSITION, PREPARATION METHOD THEREFOR, AND USE THEREOF**

(30) Priority: 16.10.2023 CN 202311336230
(71) Applicant: Hainan Tianzhuang Nutrition Engineering Co., Ltd, Haikou, Hainan 570311 (CN); West China Second University Hospital, Sichuan University, Chengdu, Sichuan 610044 (CN)
(72) Inventor: CHENG, Guo, Chengdu, Sichuan 610044 (CN); ZHANG, Lin, Chengdu, Sichuan 610044 (CN); ZHANG, Lingli, Chengdu, Sichuan 610044 (CN); ZENG, Jikai, Haikou, Hainan 570311 (CN)
(74) Representative: Altmann Stößel Dick Patentanwälte PartG mbB
(86) International application number: PCT/CN2024/097521
(87) International publication number: WO 2025/081849

(57) **Abstract**

The present invention belongs to the field of food technology, and specifically discloses a nutritional composition, a preparation method therefor, and uses thereof, wherein the nutritional composition comprises the following raw materials in the pre-determined mass ratios: 257-295 parts of proteinaceous substances, 383-395 parts of fatty substances, 632-770 parts of carbohydrates, 58-60 parts of dietary fiber substances, 4.7-5.6 parts of complexed trace minerals, 106-119 parts of complexed major minerals, 10.7-12.9 parts of multi-vitamins, 6.21-7.62 parts of choline bitartrate, 0.986-1.1 parts of L-carnitine, and 0.42-0.762 parts of taurine. The present invention provides a nutritionally complete and rationally combined nutritional composition that can comprehensively supplement the nutrients required by the individual with low immunity, thereby achieving the objectives of regulating physical functions, enhancing immunity, strengthening bones, improving constipation, promoting the recovery of bodily functions, and improving the quality of life.

## Description

### Field of the invention

The present invention belongs to the field of food technology, and specifically relates to a nutritional composition, a preparation method therefor, and uses thereof.

### Background of the invention

Immunity plays a crucial role in the body's resistance to specific pathogen infections and the prevention of disease development. Adequate and balanced nutrition serves as an effective safeguard for enhancing the body's immunity. When there is a nutritional imbalance, the immune system's function can be weakened, allowing various diseases such as infections and tumors to take advantage of the situation. While, when there is an adequate supply of nutrients, with each type of nutrient being appropriately provided and in a balanced relationship, the body's immune system is in its optimal state, leading to good human health and strong immunity. In clinical practice, the nutritional status of patients is of vital importance for the efficacy of disease treatment and health recovery. A good nutritional status helps reduce complications, lower mortality rates, improve disease prognosis, shorten hospitalization time, and reduce economic costs for patients. Some patients, due to health reasons, cannot or do not want to eat orally, thus failing to meet their nutritional needs. Especially for patients with tumors, severe infections, or major traumas, inadequate nutritional support often exacerbates bodily damage. Therefore, it is essential to provide scientific and reasonable nutritional support for patients with malnutrition or nutritional risks.

As adults age, bone formation and bone resorption become negatively balanced, with bone density reaching its peak around the age of 35 and subsequently declining, which can easily lead to fractures and even restrict or lose motor function of the trunk. Therefore, maintaining bone health and preventing osteoporosis are crucial for improving the quality of life of the adult population. Scientific and reasonable nutritional supplementation is one of the important factors affecting bone health. Calcium supplements are currently the most common method for promoting bone health, but they can also lead to adverse symptoms such as constipation. Compared to ordinary foods, nutritional compositions with clear ingredients, comprehensive nutrition, and reasonable combinations are more conducive to improving patients' immunity and strengthening bones, and are of great significance for improving malnutrition, promoting disease recovery, and enhancing the quality of life.

Patent CN101828707 discloses a nutritional composition that has the effects of enhancing immunity and preventing osteoporosis. However, it mainly promotes the biological utilization of calcium to regulate bodily functions, which may lead to the common adverse symptom of constipation in nutritional products primarily focused on calcium absorption. Therefore, it is necessary to develop a nutritionally balanced nutritional product that can enhance immunity, strengthen bones, and improve bodily functions.

### Content of the invention

To address the aforementioned issues, the present invention provides a nutritional composition that enhances immunity, strengthens bones, and/or improves constipation, which comprises the following raw materials in the pre-determined mass ratios:
257-295 parts of proteinaceous substances, 383-395 parts of fatty substances, 632-770 parts of carbohydrates, 58-60 parts of dietary fiber substances, 4.7-5.1 parts of complexed trace minerals, 106-119 parts of complexed major minerals, 10.7-12.9 parts of multi-vitamins, 6.21-7.62 parts of choline bitartrate, 0.986-1.1 parts of L-carnitine, and 0.42-0.762 parts of taurine;
the proteinaceous substance is composed of 37-47 parts of whey protein, 96-112 parts of soy protein, and 98-162 parts of casein;
the fatty substance is the microcapsulated vegetable oil powder made from 66-71 parts of medium-chain triglycerides, 62-79 parts of sunflower seed oil, 51-57 parts of soybean oil, 72-88 parts of low erucic acid rapeseed oil, and 115-117 parts of edible carriers; the edible carriers are composed of 68-70 parts of maltooligosaccharides, 31-32 parts of sodium caseinate, 3-5 parts of mono- and di-glycerides of fatty acid, 0.2-0.4 parts of vitamin E, 8-9 parts of sodium tripolyphosphate, 0.03-0.05 parts of ascorbyl palmitate, and 3-4 parts of sodium ascorbate;
the carbohydrate is composed of 420-598 parts of maltodextrin and 172-212 parts of solid corn syrup.

Further, the dietary fiber substance is composed of 18-33 parts of galactooligosaccharides and 25-42 parts of fructooligosaccharides.

Further, the complexed trace minerals are composed of 0.03-0.04 parts of copper sulfate, 0.62-0.81 parts of ferric pyrophosphate, 0.07-0.08 parts of manganese sulfate, 0.0012-0.0023 parts of sodium selenite, 0.25-0.32 parts of zinc citrate, 0.0019-0.0028 parts of potassium iodide, and 3-4 parts of edible carriers.

Further, the complexed major minerals are composed of 10.9-14.6 parts of tricalcium phosphate, 12.5-19.2 parts of calcium carbonate, 3.2-7.6 parts of magnesium carbonate, 15.1-17.6 parts of potassium chloride, 18.9-21.4 parts of potassium citrate, 32.1-32.6 parts of sodium citrate, and 7-12 parts of edible carriers.

Further, the multi-vitamins are composed of 2.68-3.00 parts of sodium L-ascorbate, 0.62-0.76 parts of vitamin E, 0.19-0.3 parts of nicotinamide, 0.1-0.2 parts of calcium D-pantothenate, 0.03-0.04 parts of pyridoxine hydrochloride, 0.04-0.06 parts of thiamine hydrochloride, 0.02-0.03 parts of vitamin A palmitate, 0.03-0.05 parts of riboflavin, 0.004-0.006 parts of folic acid, 0.001-0.003 parts of phylloquinone, 0.0003-0.0004 parts of D-biotin, 0.0001-0.0003 parts of cholecalciferol, 0.00006-0.00007 parts of cyanocobalamin, and 6-9 parts of edible carriers.

More further, the edible carrier is maltodextrin.

More further, it comprises the following raw materials in the pre-determined mass ratio:
295 parts of proteinaceous substances, 395 parts of fatty substances, 770 parts of carbohydrates, 58 parts of dietary fiber substances, 4.7 parts of complexed trace minerals, 119 parts of complexed major minerals, 10.7 parts of multi-vitamins, 7.62 parts of choline bitartrate, 0.986 parts of L-carnitine, and 0.762 parts of taurine;
the proteinaceous substance is composed of 37 parts of whey protein, 96 parts of soy protein, and 162 parts of casein;
the fatty substance is the microcapsulated vegetable oil powder made from 71 parts of medium-chain triglycerides, 62 parts of sunflower seed oil, 57 parts of soybean oil, 88 parts of low erucic acid rapeseed oil, and 117 parts of edible carriers; the edible carriers are composed of 69.16 parts of maltooligosaccharides, 32 parts of sodium caseinate, 4 parts of mono- and di-glycerides of fatty acid, 0.3 parts of vitamin E, 8.3 parts of sodium tripolyphosphate, 0.04 parts of ascorbyl palmitate, and 3.2 parts of sodium ascorbate;
the carbohydrate is composed of 598 parts of maltodextrin and 172 parts of solid corn syrup;
the dietary fiber substance is composed of 33 parts of galactooligosaccharides and 25 parts of fructooligosaccharides;
the complexed trace minerals are composed of 0.04 parts of copper sulfate, 0.62 parts of ferric pyrophosphate, 0.08 parts of manganese sulfate, 0.0012 parts of sodium selenite, 0.32 parts of zinc citrate, 0.0028 parts of potassium iodide, and 3.636 parts of maltodextrin;
the complexed major minerals are composed of 14.6 parts of tricalcium phosphate, 19.2 parts of calcium carbonate, 7.6 parts of magnesium carbonate, 15.1 parts of potassium chloride, 18.9 parts of potassium citrate, 32.1 parts of sodium citrate, and 11.5 parts of maltodextrin;
the multi-vitamins are composed of 2.68 parts of sodium L-ascorbate, 0.624 parts of vitamin E, 0.196 parts of nicotinamide, 0.12 parts of calcium D-pantothenate, 0.0336 parts of pyridoxine hydrochloride, 0.046 parts of thiamine hydrochloride, 0.02 parts of vitamin A palmitate, 0.035 parts of riboflavin, 0.0057 parts of folic acid, 0.0015 parts of phylloquinone, 0.0003 parts of D-biotin, 0.0002 parts of cholecalciferol, 0.00007 parts of cyanocobalamin, and 6.93763 parts of maltodextrin.

More further, it comprises the following raw materials in the pre-determined mass ratio:
257 parts of proteinaceous substances, 383 parts of fatty substances, 632 parts of carbohydrates, 60 parts of dietary fiber substances, 5.1 parts of complexed trace minerals, 106 parts of complexed major minerals, 12.9 parts of multi-vitamins, 6.21 parts of choline bitartrate, 1.1 parts of L-carnitine, and 0.42 parts of taurine;
the proteinaceous substance is composed of 47 parts of whey protein, 112 parts of soy protein, and 98 parts of casein;
the fatty substance is the microcapsulated vegetable oil powder made from 66 parts of medium-chain triglycerides, 79 parts of sunflower seed oil, 51 parts of soybean oil, 72 parts of low erucic acid rapeseed oil, and 115 parts of edible carriers; the edible carriers are composed of 68.66 parts of maltooligosaccharides, 31 parts of sodium caseinate, 4 parts of mono- and di-glycerides of fatty acid, 0.3 parts of vitamin E, 8.3 parts of sodium tripolyphosphate, 0.04 parts of ascorbyl palmitate, and 3 parts of sodium ascorbate;
the carbohydrate is composed of 420 parts of maltodextrin and 212 parts of solid corn syrup;
the dietary fiber substance is composed of 18 parts of galactooligosaccharides and 42 parts of fructooligosaccharides;
the complexed trace minerals are composed of 0.03 parts of copper sulfate, 0.81 parts of ferric pyrophosphate, 0.07 parts of manganese sulfate, 0.0023 parts of sodium selenite, 0.25 parts of zinc citrate, 0.0019 parts of potassium iodide, and 3.636 parts of maltodextrin;
the complexed major minerals are composed of 10.9 parts of tricalcium phosphate, 12.5 parts of calcium carbonate, 3.2 parts of magnesium carbonate, 17.6 parts of potassium chloride, 21.4 parts of potassium citrate, 32.6 parts of sodium citrate, and 7.8 parts of maltodextrin;
the multi-vitamins are composed of 3 parts of sodium L-ascorbate, 0.76 parts of vitamin E, 0.28 parts of nicotinamide, 0.12 parts of calcium D-pantothenate, 0.03 parts of pyridoxine hydrochloride, 0.05 parts of thiamine hydrochloride, 0.03 parts of vitamin A palmitate, 0.04 parts of riboflavin, 0.0042 parts of folic acid, 0.002 parts of phylloquinone, 0.0004 parts of D-biotin, 0.0001 parts of cholecalciferol, 0.00006 parts of cyanocobalamin, and 8.58324 parts of maltodextrin.

The present invention also provides a method for preparing the above nutritional composition, which comprises the following steps:
1) The raw materials are weighed according to the pre-determined ratio;
2) Choline bitartrate, L-carnitine, taurine, multi-vitamins, and complexed trace minerals are mixed, and stirred at 20-30 rpm for 10-20 min, to obtain premix 1; dietary fiber substances and whey protein are mixed, and stirred at 20-30 rpm for 10-20 min, to obtain premix 2; complexed major minerals and soy protein are mixed, and stirred at 20-30 rpm for 10-20 min, to obtain premix 3;
3) Said premix 1, premix 2, and premix 3 obtained in step 2) are mixed at 20-30 rpm for 20-30 min, to obtain total mix 1; solid corn syrup, casein, and total mix 1 are mixed at 20-30 rpm for 30-60 min, to obtain total mix 2; fatty substances, maltodextrin, and total mix 2 are mixed at 20-30 rpm for 40-60 min, to obtain the nutritional composition.

The present invention finally provides the use of the above nutritional composition in the manufacture of food or medicaments for enhancing immunity, strengthening bones, and/or improving constipation.

Further, the food or medicaments have the effects of enhancing immunity, increasing bone density, and/or promoting bowel movements.

Unless otherwise specified, said "parts" in the present invention refer to parts by mass.

In the nutritional composition of the present invention, soy protein, whey protein, and casein provide a variety of high-quality proteins, rich in amino acids with an appropriate ratio, which helps the body absorb and utilize proteins; carbohydrates are provided by solid corn syrup and maltodextrin, which have good solubility and are easily digested and absorbed by the human body; fats come from low erucic acid rapeseed oil, sunflower seed oil, medium-chain triglycerides, and soybean oil, meeting the body's needs for essential fatty acids; dietary fiber comes from galactooligosaccharides and fructooligosaccharides, which helps improve intestinal function; adding choline bitartrate promotes body fat metabolism and reduces serum cholesterol; adding L-carnitine as a carrier for fatty acid metabolism promotes the oxidation of fatty acids; adding taurine enhances the body's immunity and anti-fatigue function; adding L-ascorbic acid sodium salt, vitamin E, nicotinamide, pyridoxine hydrochloride, calcium D-pantothenate, thiamine hydrochloride, vitamin A palmitate, riboflavin, folic acid, phylloquinone, D-biotin, cholecalciferol, and cyanocobalamin provides the vitamins required by the body; adding copper sulfate, ferric pyrophosphate, manganese sulfate, sodium selenite, zinc citrate, potassium iodide, tricalcium phosphate, magnesium carbonate, calcium carbonate, potassium chloride, potassium citrate, and sodium citrate provides the minerals required by the body.

By a lot of experiments, the inventors have found that the nutritional composition obtained according to the formula of the present invention can significantly improve the health status of individuals with low immunity, enhance immunity, maintain bone health, and provide effective nutritional support for patient recovery in clinical practice. Whey protein contains a variety of essential amino acids for the human body, with a reasonable ratio, making it easy to be digested and absorbed, and the bioactive components it contains help improve immunity and restore physical fitness; casein can continuously provide slowly released protein to the human body for a long time, maintain a sense of fullness, and promote efficient absorption of constant and trace elements; soy protein is a plant-derived complete protein, rich in various essential amino acids required by the human body, easy to be digested and absorbed, and high in nutritional value; the combined use of various proteins can achieve a better ratio of amino acids, which helps to improve the bioavailability of proteins. Vitamin A plays a key role in maintaining the normal function of the immune system; vitamin E, as an important antioxidant, can protect body cells from free radical damage and delay cell aging. B vitamins, as essential nutrients for amino acid metabolism and fat metabolism in the body, participate in tissue repair and cell regeneration, promote human metabolism, regulate immune function, and enhance resistance to pathogens; vitamin C has a crucial role in antioxidation, promoting collagen synthesis, lowering cholesterol, and participating in body detoxification, as well as helps reduce the harm caused by medicaments and environmental pollution to the human body, and moreover, it participates in the metabolism of proteins and collagen aminopolysaccharides in bone tissue, facilitating calcium absorption and deposition into bones; vitamin D is beneficial for regulating bone calcium absorption and promoting bone formation. Zinc, a mineral, is added to participate in the synthesis and activity of various enzymes in the body and contributes to immune function; ferrum is an essential component of hemoglobin, playing a crucial role in maintaining normal hematopoiesis, and is also a vital component of many enzymes and immune system compounds, enhancing the body's immunity and resistance to diseases; selenium not only boosts the body's immunity and maintains the normal function of vital organs such as the heart, liver, lungs, and stomach, but also protects and repairs cells, enhancing the body's antioxidant capacity; calcium is used to maintain the physical strength of human bones and serves as a fundamental guarantee for bone health. L-carnitine, as an important substance involved in fat metabolism, can promote fat conversion, improve fat utilization, reduce fat accumulation in the body, and effectively regulate intracellular energy metabolism; choline helps maintain the integrity of cell membranes, improve fat absorption and utilization, protect the liver, etc; taurine exerts a key role in regulating neural conduction, modulating lipid digestion and absorption, and enhancing immune function, making it an essential active substance for regulating normal physiological activities of the body; the addition of galactooligosaccharides and fructooligosaccharides serves as dietary fiber, maintaining intestinal immune function while preventing and alleviating constipation symptoms.

The nutritional composition of the present invention, which enhances immunity and strengthens bones, provides comprehensive supplementation of essential nutrients for individuals with low immunity by specific ingredients and mass ratios, so as to regulate bodily functions, enhance immunity, strengthen bones, improve constipation, promote the recovery of bodily functions, and enhance the quality of life. It is a nutritionally comprehensive and rationally formulated nutritional composition, with actual promotional values.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. Changes in mouse body weight;
Figure 2. The effect of nutritional composition on immunosuppressed mice (A: the effect on body weight, B: The effect on food intake; * indicates *P* < 0.05 compared to the model group).
Figure 3. The effect of nutritional composition on serum TNF-α, IgG, and IgM concentrations in mice (A: TNF-α concentration, B: IgG concentration, C: IgM concentration, * indicates *P* < 0.05 compared to the model group).
Figure 4. The effect of nutritional composition on the frequency of defecation and the number of fecal pellets in mice (A: The frequency of defecation, B: The number of fecal pellets, * indicates *P* < 0.05 compared to the model group).

### Examples

The raw materials and equipment in the specific examples of the present invention can be obtained by purchasing those commercially available, wherein low erucic acid rapeseed oil is rapeseed oil with an erucic acid content of not exceeding 3% based on the fatty acid composition; maltooligosaccharide is an oligosaccharide composed of more than 3 and less than 10 glucoses linked by α-1,4 glycosidic bonds.

### Example 1: Preparation of the nutritional composition according to the present invention

Formula: 295 parts of proteins, comprising 37 parts of whey proteins, 96 parts of soy proteins, and 162 parts of casein;
395 parts of microcapsulated vegetable oil powders: which is a mixture made from 71 parts of medium-chain triglycerides, 62 parts of sunflower seed oils, 57 parts of soybean oils, 88 parts of low erucic acid rapeseed oils, as well as the edible carriers, including 69.16 parts of maltooligosaccharides, 32 parts of sodium caseinate, 4 parts of mono- and di-glycerides of fatty acid, 0.3 parts of vitamin E, 8.3 parts of sodium tripolyphosphate, 0.04 parts of ascorbyl palmitate, and 3.2 parts of sodium ascorbate;
770 parts of carbohydrates, comprising 598 parts of maltodextrin and 172 parts of solid corn syrup;
58 parts of dietary fibers, comprising 33 parts of galactooligosaccharides and 25 parts of fructooligosaccharides.
10.7 parts of multi-vitamins: which is a mixture made from 2.68 parts of sodium L-ascorbate, 0.624 parts of vitamin E, 0.196 parts of nicotinamide, 0.12 parts of calcium D-pantothenate, 0.0336 parts of pyridoxine hydrochloride, 0.046 parts of thiamine hydrochloride, 0.02 parts of vitamin A palmitate, 0.035 parts of riboflavin, 0.0057 parts of folic acid, 0.0015 parts of phylloquinone, 0.0003 parts of D-biotin, 0.0002 parts of cholecalciferol, 0.00007 parts of cyanocobalamin, and the remaining parts of maltodextrin as the carrier of multi-vitamins;
4.7 parts of complexed trace minerals: which is made by mixing 0.04 parts of copper sulfate, 0.62 parts of ferric pyrophosphate, 0.08 parts of manganese sulfate, 0.0012 parts of sodium selenite, 0.32 parts of zinc citrate, 0.0028 parts of potassium iodide, and the remaining parts of maltodextrin as the carrier of complexed trace minerals;
119 parts of complexed major minerals: which is made by mixing 14.6 parts of tricalcium phosphate, 19.2 parts of calcium carbonate, 7.6 parts of magnesium carbonate, 15.1 parts of potassium chloride, 18.9 parts of potassium citrate, 32.1 parts of sodium citrate, and the remaining parts of maltodextrin as the carrier of complexed major minerals;
7.62 parts of choline bitartrate; 0.986 parts of L-carnitine; 0.762 parts of taurine.

### Preparation method:

(1) The raw materials were accurately weighed and divided into 5 groups based on the order of increasing mass ratio;
   Raw material group 1: choline bitartrate, L-carnitine, taurine, multi-vitamins, and complexed trace minerals;
   Raw material group 2: dietary fiber and whey protein;
   Raw material group 3: complexed trace minerals and soy protein;
   Raw material group 4: solid corn syrup and casein;
   Raw material group 5: microcapsulated vegetable oil powders and maltodextrin.
(2) Premixing raw materials with low amounts
   Premix 1: Raw material group 1 was transferred to a pre-mixer, and mixed at 25 rpm for 15 min to obtain premix 1;
   Premix 2: Premix 1 was taken out, and then raw material group 2 was transferred to the pre-mixer, and mixed at 25 rpm for 15 min to obtain premix 2;
   Premix 3: Premix 2 was taken out, and then raw material group 3 was transferred to the pre-mixer, and mixed at 25 rpm for 15 min to obtain premix 3.
(3) Total mix
   Total mix 1: Premixes 1, 2, and 3 were transferred to a total mixer, and then mixed at 30 rpm for 30 min to obtain total mix 1;
   Total mix 2: Premix 4 was transferred to the total mixer, and then mixed with total mix 1 at 30 rpm for 40 min to obtain total mix 2;
   Total mix 3: Premix 5 was transferred to the total mixer, and then mixed with total mix 2 at 30 rpm for 60 min to obtain the finished product;
(4) Sub-packaging and packaging

The mixed finished product was sub-packaged and packaged under sterile conditions, and all the above procedures were carried out in a clean and dry environment.

### Example 2: Preparation of the nutritional composition according to the present invention

Formula: 257 parts of proteins, comprising 47 parts of whey proteins, 112 parts of soy proteins, and 98 parts of casein;
383 parts of microcapsulated vegetable oil powders: which is a mixture made from 66 parts of medium-chain triglycerides, 79 parts of sunflower seed oils, 51 parts of soybean oils, 72 parts of low erucic acid rapeseed oils, as well as the edible carriers, comprising 68.66 parts of maltooligosaccharide, 31 parts of sodium caseinate, 4 parts of mono- and di-glycerides of fatty acid, 0.3 parts of vitamin E, 8 parts of sodium tripolyphosphate, 0.04 parts of ascorbyl palmitate, and 3 parts of sodium ascorbate;
632 parts of carbohydrates, comprising 420 parts of maltodextrin and 212 parts of solid corn syrup;
60 parts of dietary fibers, comprising 18 parts of galactooligosaccharides and 42 parts of fructooligosaccharides.
12.9 parts of multi-vitamins: which is made by mixing 3 parts of sodium L-ascorbate, 0.76 parts of vitamin E, 0.28 parts of nicotinamide, 0.12 parts of calcium D-pantothenate, 0.03 parts of pyridoxine hydrochloride, 0.05 parts of thiamine hydrochloride, 0.03 parts of vitamin A palmitate, 0.04 parts of riboflavin, 0.0042 parts of folic acid, 0.002 parts of phylloquinone, 0.0004 parts of D-biotin, 0.0001 parts of cholecalciferol, 0.00006 parts of cyanocobalamin, and the remaining parts of maltodextrin as the carrier of multi-vitamins;
5.1 parts of complexed trace minerals: which is made by mixing 0.03 parts of copper sulfate, 0.81 parts of ferric pyrophosphate, 0.07 parts of manganese sulfate, 0.0023 parts of sodium selenite, 0.25 parts of zinc citrate, 0.0019 parts of potassium iodide, and the remaining parts of maltodextrin as the carrier of complexed trace minerals;
106 parts of complexed major minerals: which is made by mixing 10.9 parts of tricalcium phosphate, 12.5 parts of calcium carbonate, 3.2 parts of magnesium carbonate, 17.6 parts of potassium chloride, 21.4 parts of potassium citrate, 32.6 parts of sodium citrate, and the remaining parts of maltodextrin as the carrier of complexed major minerals;
6.21 parts of choline bitartrate; 1.1 parts of L-carnitine; 0.42 parts of taurine.

### Preparation method:

(1) The raw materials were accurately weighed and divided into 5 groups based on the order of increasing mass ratio;
   Raw material group 1: choline bitartrate, L-carnitine, taurine, multi-vitamins, and complexed trace minerals;
   Raw material group 2: dietary fiber and whey protein;
   Raw material group 3: complexed trace minerals and soy protein;
   Raw material group 4: solid corn syrup and casein;
   Raw material group 5: microcapsulated vegetable oil powders and maltodextrin.
(2) Premixing raw materials with low amounts
   Premix 1: Raw material group 1 was transferred to a pre-mixer, and mixed at 30 rpm for 20 min to obtain premix 1;
   Premix 2: Premix 1 was taken out, and then raw material group 2 was transferred to the pre-mixer, and mixed at 30 rpm for 20 min to obtain premix 2;
   Premix 3: Premix 2 was taken out, and then raw material group 3 was transferred to the pre-mixer, and mixed at 30 rpm for 20 min to obtain premix 3.
(3) Total mix
   Total mix 1: Premixes 1, 2, and 3 were transferred to a total mixer, and then mixed at 30 rpm for 30 min to obtain total mix 1;
   Total mix 2: Premix 4 was transferred to the total mixer, and then mixed with total mix 1 at 30 rpm for 60 min to obtain total mix 2;
   Total mix 3: Premix 5 was transferred to the total mixer, and then mixed with total mix 2 at 30 rpm for 60 min to obtain the finished product;
(4) Sub-packaging and packaging

The mixed finished product was sub-packaged and packaged under sterile conditions, and all the above procedures were carried out in a clean and dry environment.

### Comparative example 3

Other conditions and procedures were the same as in Example 1, with the difference being that the amount of casein in the protein was adjusted to 40 parts, and the total amount of proteinaceous substances was changed accordingly; the amount of microcapsulated vegetable oil powders was adjusted to 220 parts (comprising 27 parts of medium-chain triglycerides, 32 parts of sunflower seed oil, 48 parts of soybean oil, 47 parts of low erucic acid rapeseed oil, and the rest consisting of edible carrier, comprising 33 parts of maltooligosaccharide, 16 parts of sodium caseinate, 2.5 parts of mono- and di-glycerides of fatty acid, 3.3 parts of sodium tripolyphosphate, without adding vitamin E, ascorbyl palmitate, and sodium ascorbate); in the complexed major minerals, tricalcium phosphate was omitted, and the amount of calcium carbonate was adjusted to 2.1 parts, with the total amount of complexed major minerals changing accordingly.

### Comparative example 4

Other conditions and procedures were the same as in Example 1, with the difference being that in the protein, the amount of whey protein was adjusted to 12 parts, while the amount of casein was adjusted to 40 parts, and the total amount of proteinaceous substances was changed accordingly; taurine was omitted; in the edible carriers of microcapsulated vegetable oil powders, the amount of vitamin E was adjusted to 0.02 parts, while the amount of ascorbyl palmitate was adjusted to 0.01 parts, and sodium ascorbate was eliminated, with the total amount of microcapsulated vegetable oil powders changing accordingly; the amount of cholecalciferol in the multi-vitamins was adjusted to 0.00003 parts, and the total amount of multi-vitamins was changed accordingly.

### Comparative example 5

Other conditions and procedures were the same as in Example 2, with the difference being that whey protein was omitted from the protein, the amount of casein was adjusted to 88 parts, the amount of soy protein was adjusted to 131 parts, the amount of taurine was adjusted to 0.02 parts, and the total amount of protein substances was changed accordingly; in the edible carriers of microcapsulated vegetable oil powders, vitamin E was omitted, the amount of ascorbyl palmitate was adjusted to 0.008 parts, the amount of sodium ascorbate was adjusted to 0.2 parts, and the total amount of microcapsulated vegetable oil powders was changed accordingly; in the complexed major minerals, the amount of tricalcium phosphate was adjusted to 1.6 parts; fructooligosaccharide was omitted from dietary fiber, and the total amount of complexed major minerals was changed accordingly.

### Comparative example 6

Other conditions and procedures were the same as in Example 2, with the difference being that the amount of whey protein in the protein was adjusted to 61 parts, and the total amount of proteinaceous substances was changed accordingly; taurine was deleted; vitamin E and ascorbyl palmitate were omitted from the edible carrier of the microcapsulated vegetable oil powders, and the amount of sodium ascorbate was adjusted to 0.7 parts, with the total amount of microcapsulated vegetable oil powders changing accordingly; the amount of galactooligosaccharide in dietary fiber was adjusted to 2 parts, and the amount of fructooligosaccharide was adjusted to 3 parts, with the total amount of dietary fiber changing accordingly.

The beneficial effects of the present invention were further illustrated with reference to the following experimental examples:

### Experimental example 1: Performance testing of the nutritional composition according to the present invention

### I. Stability test

The nutritional compositions prepared in Examples 1-2 and Comparative Examples 3-6 were stored at a temperature of 40 ± 2 °C and a relative humidity of 75±5% for 3 months. Samples were taken at the beginning of the experiment, and at the end of the first, second, and third months. The peroxide value was tested using titration method, and the results are shown in Table 1.

**Table 1. Results of accelerated stability test.**

| Detection time (Month) | Peroxide value (meq/kg) | | | | | |
|---|---|---|---|---|---|---|
| | Example 1 | Example 2 | Comparative example 3 | Comparative example 4 | Comparative example 5 | Comparative example 6 |
| 0 | 1.11 | 1.12 | 1.40 | 1.30 | 1.32 | 1.29 |
| 1 | 1.59 | 1.62 | 4.60 | 3.66 | 4.20 | 3.20 |
| 2 | 2.21 | 2.30 | 9.20 | 6.32 | 8.60 | 8.40 |
| 3 | 3.65 | 3.72 | 11.8 | 9.48 | 10.35 | 9.98 |

The above stability results indicated that under the conditions of temperature 40±2 °C and humidity 75±5%, the composition that was not prepared according to the preferred range, i.e., Examples 1-2, showed a rapid increase in peroxide values as the testing time progressed, approaching or exceeding 10 meq/kg after 3 months, that indicated a certain risk in its stability. However, the nutritional composition that was added and mixed with tocopherol concentrate, sodium ascorbate, and ascorbyl palmitate simultaneously, and the added amount was within the preferred range, i.e., Examples 1-2, had the best antioxidant effect, indicating the best stability.

### II. Antioxidant activity test

The nutritional compositions prepared in Examples 1-2 and Comparative Examples 3-6 were tested for their DPPH radical scavenging rates, hydroxyl radical scavenging rates, and superoxide anion scavenging rates using the DPPH radical scavenging assay, salicylic acid assay, and pyrogallol autoxidation method, respectively. The antioxidant activity of the nutritional compositions was evaluated, and the results are shown in Table 2.

**Table 2. Antioxidant activity of each example.**

| Groups | DPPH radical scavenging rate | hydroxyl radical scavenging rate | superoxide anion scavenging rate |
|---|---|---|---|
| Example 1 | 92.29%±5.14 | 62.14%±2.41 | 70.12%±3.16 |
| Example 2 | 89.57%±3.47 | 60.25%±3.02 | 71.56%±2.47 |
| Comparative example 3 | 75.67%±4.11 | 52.87%±2.58 | 53.47%±1.78 |
| Comparative example 4 | 70.68%±3.58 | 43.25%±2.32 | 42.93%±2.04 |
| Comparative example 5 | 66.24%±4.01 | 35.70%±2.91 | 32.30%±1.55 |
| Comparative example 6 | 60.11%±3.39 | 30.33%±1.88 | 30.24±±2.03 |

DPPH was a relatively stable nitrogen-containing organic radical, and commonly used as an indicator to measure the antioxidant activities of samples; hydroxyl radicals were the most harmful free radicals, and the ability to scavenge hydroxyl radicals was a good proof of antioxidant capacity; and superoxide anion radicals were prone to inducing lipid peroxidation reactions in organisms. As shown in Table 2, the DPPH radical scavenging rates of Examples 1 and 2 were 92.29% and 89.57%, respectively; the hydroxyl radical scavenging rates were 62.14% and 60.25%, respectively; and the superoxide anion scavenging rates were 70.12% and 71.56%, respectively, which were much higher than those of other examples. This indicated that Examples 1 and 2 had better antioxidant activities, and also indicated that the antioxidant capacity of the nutritional compositions obtained in Examples 1 and 2 had a synergistic effect.

### III. Toxicity test

The nutritional compositions prepared in Examples 1-2 and Comparative Examples 3-6 were tested for acute toxicity in normal mice.

35 male SPF-grade Kunming mice, aged 4 weeks and weighing 20±2 g, were selected. Before the formal experiment, the mice underwent a 3-day acclimatization period. During the experiment, the environmental temperature was set at 25±1 °C, and the humidity at 40%±10%, with 12 hours of light exposure and 12 hours of darkness. The experimental mice were divided into 7 groups: a blank control group, Examples 1-2 groups, and Comparative Examples 3-6 groups, for acute toxicity testing. The mice were randomly assigned to 7 cages based on body weight, with 5 mice per cage. The experimental groups were administered with the nutritional compositions obtained in Examples 1-2 and Comparative Examples 3-6 at a maximum gavage dose of 5 g/(kg·d), respectively; the blank control group was administered with an equivalent dose of saline. All mice in each group were allowed to feed freely except for gavage. After gavage, changes in the appearance, behavior, and secretions of the mice were observed daily for 14 consecutive days. Animal body weights were weighed every other day. During the trial period, animals that showed moderate mortality or were moribund were immediately subjected to autopsy examination. Surviving animals were euthanized and subjected to autopsy examination after the completion of gavage.

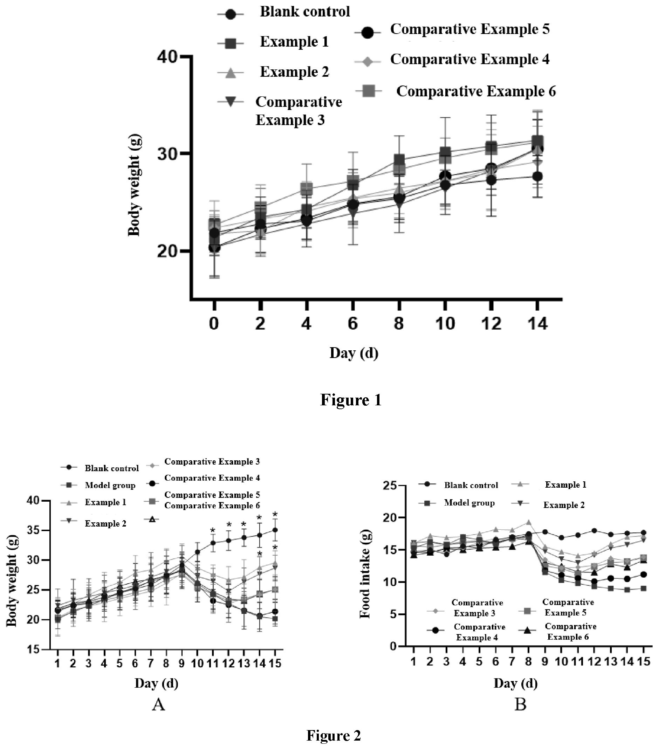

Detection indicators: 1. Changes in mouse body weight; 2. Acute injury conditions of mouse tissues and organs. The results are shown in Figure 1 and Table 3.

**Table 3. Results of mouse organ testing.**

| Groups | Heart | Liver | Kidney | Spleen | Thymus | Lung | Small intestine | Stomach |
|---|---|---|---|---|---|---|---|---|
| Blank control group | Normal | Normal | Normal | Normal | Normal | Normal | Normal | Normal |
| Example 1 group | Normal | Normal | Normal | Normal | Normal | Normal | Normal | Normal |
| Example 2 group | Normal | Normal | Normal | Normal | Normal | Normal | Normal | Normal |
| Comparative example 3 group | Normal | Normal | Normal | Normal | Normal | Normal | Normal | Normal |
| Comparative example 4 group | Normal | Normal | Normal | Normal | Normal | Normal | Normal | Normal |
| Comparative example 5 group | Normal | Normal | Normal | Normal | Normal | Normal | Normal | Normal |
| Comparative example 6 group | Normal | Normal | Normal | Normal | Normal | Normal | Normal | Normal |

As shown in Figure 1, the weight of the mice in the blank control group, Examples 1-2 groups, and Comparative examples 3-6 groups all showed a normal growth trend, and after the start of gavage, the weight gain in each Example group was consistently higher than that of the control group, indicating that Examples 1-2 and Comparative Examples 3-6 could promote weight gain in mice, without affecting their normal developmental status.

After two consecutive weeks of gavage feeding, the anatomical examination results showed that compared with the blank control group, there were no abnormalities in the texture and color of the organs in each experimental group. This indicated that the test compounds obtained in Examples 1-2 as well as Comparative Examples 3-6, did not cause visible damage to the organs of mice, and there was no acute toxic injury.

### IV. Immune effect test

The nutritional compositions prepared in Examples 1-2 and Comparative Examples 3-6 were tested for their immune response in immunocompromised mice.

Forty 4-week-old male Kunming mice of SPF grade, weighing 20±2 g, were selected. Before the formal experiment, the mice underwent a 3-day adaptive period. During the experiment, the environmental temperature was set at 25±1 °C, and the humidity at 40%±10%, with a 12-hour light period and a 12-hour dark period. The experimental mice were randomly divided into 8 cages based on body weight, with 5 mice for one cage, and were assigned to a blank control group, a model group, and Examples 1-6 groups, that is, 8 groups for immune response testing. Examples 1-2 groups and Comparative Examples 3-6 groups were administered with a suspension of Examples 1-2 and Comparative Examples 3-6 at 500 mg/(kg·d) via gavage, respectively, while the blank control group and the model group were administered with an equivalent dose of saline via gavage. Starting from day 9, except for the blank control group, mice in other groups were intraperitoneally injected with cyclophosphamide (CTX) solution at 80 mg/(kg·d) to establish an immunosuppression model, which lasted for three consecutive days. During this period, the gavage was maintained. On day 12 of the experiment, the intraperitoneal injection of CTX was stopped, and the mice were continuously fed until day 15. The body weight was weighed and recorded before gavage. The average food intake of mice in each group was calculated by weighing the feed weight daily. Before the end of the experiment, three mice were subjected to fasting without water deprivation. Mice were euthanized by cervical dislocation, and their eyeballs were extracted, blood was collected, and then the thymus, spleen, liver, and kidneys were dissected and weighed to obtain wet weight.

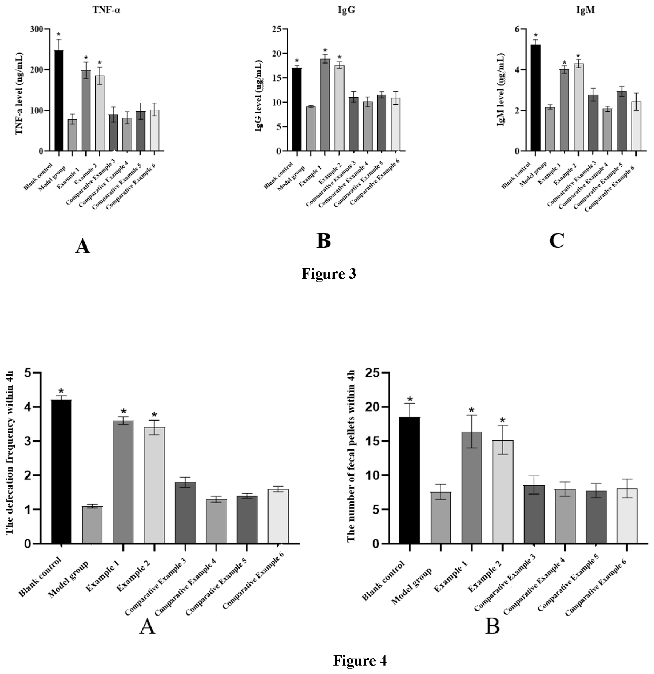

Detection indicators: 1. Changes in mouse weight; 2. Changes in mouse food intake; 3. Mouse organ index; 4. Levels of serum immune factors and immunoglobulins in mice. The results are shown in Figure 2, Table 4, and Figure 3.

As shown in Figures 2A and 2B, during the first 8 days, mice in all groups maintained their feeding and weight gain, with no significant differences in food intake and weight among them. Starting from day 9, CTX was administered to establish an immunocompromised mouse model for 3 consecutive days. During this period, except for the blank control group, the body weight of mice in the other groups decreased, and their free food intake significantly decreased, indicating successful modeling. After stopping the modeling process from day 12, the body weight of mice in the other groups, except for the model group, recovered to some extent, but it could not reach the body weight level of the blank control group. Compared to the model group, the body weight of mice in Examples 1 and 2 groups significantly increased from day 12 to day 15 (*P* < 0.05), and the food intake of mice in Examples 1-2 groups gradually returned to the pre-modeling level. During the period from day 12 to day 15, although the body weight and food intake of mice in Comparative Examples 3, 5, and 6 groups increased, there was no statistically significant difference compared to the mice in the model group (P>0.05). However, from day 9 to day 15, the body weight and food intake of mice in Comparative Example 4 group showed a downward trend. Therefore, the experimental results indicated that during days 1 to 8 of the experiment, the body weight of mice in each group showed an increasing trend. During the modeling period from Day 9 to Day 11, CTX not only suppressed the immune system, but also affected other physiological activities of mice, such as reduced food intake and hindered growth performance. However, after interventions in Examples 1-2 groups and Comparative Examples 3-6 groups, Examples 1-2 could effectively maintain the body weight and food intake of mice. This indicated that Examples 1 and 2 were helpful for the recovery of body weight in mice with low immunity, while Comparative Examples 3-6 did not significantly improve the situation of reduced body weight and food intake in mice with low immunity.

**Table 4. Effects of nutritional compositions on organ indices in immunocompromised mice.**

| Groups | Kidney index | Liver index | Spleen index | Thymus index |
|---|---|---|---|---|
| Blank group | 1.4056±0.1009 | 5.4678±0.1998 | 0.3214±0.0382^{a} | 0.2980±0.0547^{a} |
| Model group | 1.2298±0.3574 | 5.4439±0.3281 | 0.1547±0.0551 | 0.0914±0.0382 |
| Example 1 | 1.3648±0.1964 | 5.5324±0.2472 | 0.2571±0.0745^{a} | 0.2424±0.0541^{a} |
| Example 2 | 1.3354±0.1563 | 5.3248±0.3364 | 0.2475±0.0324^{a} | 0.2208±0.1540^{a} |
| Comparative Example 3 | 1.3036±0.1987 | 5.3477±0.5731 | 0.1764±0.0471 | 0.1218±0.0381 |
| Comparative Example 4 | 1.3221±0.2547 | 5.1584±0.3491 | 0.1589±0.0457 | 0.1038±0.0355 |
| Comparative Example 5 | 1.3248±0.1274 | 5.4568±0.5781 | 0.1848±0.3338 | 0.1178±0.0282 |
| Comparative Example 6 | 1.3154±0.1489 | 5.2547±0.3258 | 0.1762±0.7813 | 0.1289±0.0450 |

| | | | | |
|---|---|---|---|---|
| Note: a indicates *P* < 0.05 compared with the model group | | | | |

As shown in Table 4, there were no significant differences in liver index and kidney index among the mice groups. However, regarding the spleen and thymus, which were the main immune organs, the spleen index and thymus index of the blank group, Example 1, and Example 2 were significantly higher than those of the model group (*P*<0.05), indicating that Example 1 and Example 2 contributed to the recovery of the immune organs in mice, thereby enhancing immunity. Compared with the model group, there were no significant differences in spleen index and kidney index among the mice in Comparative Examples 3-6 groups (*P*>0.05). The experimental results showed that Example 1 and Example 2 could significantly improve the recovery of immune organs in immunocompromised mice.

TNF-α was an important inflammatory cytokine that regulated various immune and inflammatory responses, aiding the body in resisting pathogen infection and performing other crucial physiological functions. IgG and IgM were the primary immunoglobulins, possessing multiple immune regulatory functions. As shown in Figure 3A, compared to the model group, the serum TNF-α concentrations in the blank control group as well as Examples 1 and 2 groups were significantly increased (*P*<0.05). Figures 3B and 3C demonstrated that, compared to the model group, the serum IgG and IgM concentrations in the blank control group, Example 1 group, and Example 2 group were significantly increased (P<0.05). The experimental results indicated that Examples 1 and 2 could significantly increase the levels of serum immune factors TNF-α and immunoglobulins IgG and IgM, thereby enhancing the body's immune capacity.

### V. Calcium absorption test

The nutritional compositions prepared in Examples 1-2 and Comparative Examples 3-6 were tested for their effect on promoting calcium absorption in immunocompromised mice. The steps were as follows:
The feeding and intervention methods for mice were the same as described in "IV. Immune effect test". After the intervention, three mice were selected from each group for eyeball extraction and blood sampling. Enzyme-linked immunosorbent assay (ELISA) kits were used to detect the serum levels of 25-hydroxyvitamin D and osteocalcin in mice of each group. The results are shown in Table 5.

**Table 5. The effect of nutritional composition on the levels of serum 25-hydroxyvitamin D and osteocalcin in mice.**

| Groups | 25-hydroxyvitamin D | Osteocalcin |
|---|---|---|
| Blank group | 54.53±4.61^{a} | 6.67±0.75^{a} |
| Model group | 35.56±3.57 | 3.49±0.43 |
| Example 1 | 50.24±3.38^{a} | 6.04±0.55^{a} |
| Example 2 | 49.65±2.47^{a} | 5.87±0.47^{a} |
| Comparative Example 3 | 36.47±3.58 | 3.45±0.36 |
| Comparative Example 4 | 32.63±3.04 | 3.21±0.35 |
| Comparative Example 5 | 33.98±2.84 | 3.44±0.28 |
| Comparative Example 6 | 32.45±2.21 | 3.43±0.32 |

| | | |
|---|---|---|
| Note: a indicates *P* < 0.05 compared with the model group | | |

The primary function of vitamin D was to regulate calcium and phosphorus metabolism in the body, reducing the risk of osteoporosis and fractures. 25-hydroxyvitamin D served as a reliable indicator of vitamin D levels in the human body. Osteocalcin, a non-collagen protein produced and secreted by osteoblasts, served as a bone metabolic marker, and could directly reflect osteocyte activity.

As shown in Table 5, compared with the model group, the levels of serum 25-hydroxyvitamin D and osteocalcin in the blank group, Example 1 group, and Example 2 group were significantly increased (*P*<0.05), indicating that Example 1 and Example 2 could promote bone health and strengthen bones by promoting calcium absorption, increasing osteocalcin levels, and enhancing bone density.

### VI. Test of laxative effect

The nutritional compositions prepared in Examples 1-2 and Comparative Examples 3-6 were tested for their laxative effect on immunocompromised mice, and the steps were as follows:
The feeding and intervention methods for mice were the same as described in "IV. Immune effect test". After the intervention, 5 mice from each group were placed in metabolic cages, and the number of defecations within 4 hours was counted. Feces were collected and the number of fecal pellets was recorded. The results are shown in Figure 4.

Low immunity could lead to slow intestinal peristalsis and delayed excretion of intestinal contents, seriously affecting the quality of life. Nutritional compositions had the effect of improving small intestinal transit efficiency and promoting intestinal peristalsis. By observing the defecation frequency and the number of fecal pellets in mice within 4 hours, the degree of laxative effect of nutritional compositions could be evaluated.

From Figure 4A, compared with the model group, the number of bowel movements within 4 hours significantly increased in the mice from the blank control group, Example 1 group, and Example 2 group (P<0.05); as shown in Figure 4B, compared with the model group, the number of fecal pellets in the mice from the blank control group, Example 1 group, and Example 2 group significantly increased (*P*<0.05). The experimental results showed that Examples 1 and 2 could significantly improve the small intestine transmit efficiency, increase the frequency of defecation, and improve constipation in mice.

The above experimental results indicated that by administering the nutritional compositions obtained in Examples 1-2 and Comparative Examples 3-6 to mice via gavage at the maximum dose using the maximum tolerance method, and observing the body weight and various internal organs of the mice, it was verified that Examples 1-2 and Comparative Examples 3-6 were safe and non-toxic, with no visible damage to the organs, and did not affect the normal growth and development status of the mice. The immune effect test had demonstrated that Examples 1-2 could significantly improve the immunosuppressive effect induced by CTX, helping to slow down the weight loss and reduced food intake induced by immunosuppression, and accelerate the recovery of immune organs such as the spleen and thymus. Meanwhile, Examples 1-2 had the effect of promoting calcium absorption in the body, increasing osteocalcin concentration, enhancing bone density, and strengthening bones. In addition, Examples 1-2 could also increase the frequency of defecation in mice and improve constipation in mice.

In summary, the nutritional composition of the present invention has the effects of enhancing immunity, increasing bone density, and promoting bowel movements. Thereby, it could comprehensively supplement the nutrients required by the individuals with low immunity, and achieve the goals of enhancing immunity, strengthening bones, improving constipation, promoting the restoration of normal bodily functions, and improving the quality of life.

## Claims

1. A nutritional composition for enhancing immunity, strengthening bones, and/or improving constipation, **characterized in that** it comprises the following raw materials in the pre-determined mass ratio:
257-295 parts of proteinaceous substances, 383-395 parts of fatty substances, 632-770 parts of carbohydrates, 58-60 parts of dietary fiber substances, 4.7-5.1 parts of complexed trace minerals, 106-119 parts of complexed major minerals, 10.7-12.9 parts of multi-vitamins, 6.21-7.62 parts of choline bitartrate, 0.986-1.1 parts of L-carnitine, and 0.42-0.762 parts of taurine;
the proteinaceous substance is composed of 37-47 parts of whey protein, 96-112 parts of soy protein, and 98-162 parts of casein;
the fatty substance is the microcapsulated vegetable oil powder made from 66-71 parts of medium-chain triglycerides, 62-79 parts of sunflower seed oil, 51-57 parts of soybean oil, 72-88 parts of low erucic acid rapeseed oil, and 115-117 parts of edible carriers; the edible carriers are composed of 68-70 parts of maltooligosaccharides, 31-32 parts of sodium caseinate, 3-5 parts of mono- and di-glycerides of fatty acid, 0.2-0.4 parts of vitamin E, 8-9 parts of sodium tripolyphosphate, 0.03-0.05 parts of ascorbyl palmitate, and 3-4 parts of sodium ascorbate;
the carbohydrate is composed of 420-598 parts of maltodextrin and 172-212 parts of solid corn syrup.

2. The nutritional composition according to claim 1, **characterized in that** the dietary fiber substance is composed of 18-33 parts of galactooligosaccharides and 25-42 parts of fructooligosaccharides.

3. The nutritional composition according to claim 1, **characterized in that** the complexed trace minerals are composed of 0.03-0.04 parts of copper sulfate, 0.62-0.81 parts of ferric pyrophosphate, 0.07-0.08 parts of manganese sulfate, 0.0012-0.0023 parts of sodium selenite, 0.25-0.32 parts of zinc citrate, 0.0019-0.0028 parts of potassium iodide, and 3-4 parts of maltodextrin.

4. The nutritional composition according to claim 1, **characterized in that** the complexed major minerals are composed of 10.9-14.6 parts of tricalcium phosphate, 12.5-19.2 parts of calcium carbonate, 3.2-7.6 parts of magnesium carbonate, 15.1-17.6 parts of potassium chloride, 18.9-21.4 parts of potassium citrate, 32.1-32.6 parts of sodium citrate, and 7-12 parts of maltodextrin.

5. The nutritional composition according to claim 1, **characterized in that** the multi-vitamins are composed of 2.68-3.00 parts of sodium L-ascorbate, 0.62-0.76 parts of vitamin E, 0.19-0.3 parts of nicotinamide, 0.1-0.2 parts of calcium D-pantothenate, 0.03-0.04 parts of pyridoxine hydrochloride, 0.04-0.06 parts of thiamine hydrochloride, 0.02-0.03 parts of vitamin A palmitate, 0.03-0.05 parts of riboflavin, 0.004-0.006 parts of folic acid, 0.001-0.003 parts of phylloquinone, 0.0003-0.0004 parts of D-biotin, 0.0001-0.0003 parts of cholecalciferol, 0.00006-0.00007 parts of cyanocobalamin, and 6-9 parts of maltodextrin.

6. The nutritional composition according to claim 1, **characterized in that** it comprises the following raw materials in the pre-determined mass ratio:
295 parts of proteinaceous substances, 395 parts of fatty substances, 770 parts of carbohydrates, 58 parts of dietary fiber substances, 4.7 parts of complexed trace minerals, 119 parts of complexed major minerals, 10.7 parts of multi-vitamins, 7.62 parts of choline bitartrate, 0.986 parts of L-carnitine, and 0.762 parts of taurine;
the proteinaceous substance is composed of 37 parts of whey protein, 96 parts of soy protein, and 162 parts of casein;
the fatty substance is the microcapsulated vegetable oil powder made from 71 parts of medium-chain triglycerides, 62 parts of sunflower seed oil, 57 parts of soybean oil, 88 parts of low erucic acid rapeseed oil, and 117 parts of edible carriers; the edible carriers are composed of 69.16 parts of maltooligosaccharides, 32 parts of sodium caseinate, 4 parts of mono- and di-glycerides of fatty acid, 0.3 parts of vitamin E, 8.3 parts of sodium tripolyphosphate, 0.04 parts of ascorbyl palmitate, and 3.2 parts of sodium ascorbate;
the carbohydrate is composed of 598 parts of maltodextrin and 172 parts of solid corn syrup;
the dietary fiber substance is composed of 33 parts of galactooligosaccharides and 25 parts of fructooligosaccharides;
the complexed trace minerals are composed of 0.04 parts of copper sulfate, 0.62 parts of ferric pyrophosphate, 0.08 parts of manganese sulfate, 0.0012 parts of sodium selenite, 0.32 parts of zinc citrate, 0.0028 parts of potassium iodide, and 3.636 parts of maltodextrin;
the complexed major minerals are composed of 14.6 parts of tricalcium phosphate, 19.2 parts of calcium carbonate, 7.6 parts of magnesium carbonate, 15.1 parts of potassium chloride, 18.9 parts of potassium citrate, 32.1 parts of sodium citrate, and 11.5 parts of maltodextrin;
the multi-vitamins are composed of 2.68 parts of sodium L-ascorbate, 0.624 parts of vitamin E, 0.196 parts of nicotinamide, 0.12 parts of calcium D-pantothenate, 0.0336 parts of pyridoxine hydrochloride, 0.046 parts of thiamine hydrochloride, 0.02 parts of vitamin A palmitate, 0.035 parts of riboflavin, 0.0057 parts of folic acid, 0.0015 parts of phylloquinone, 0.0003 parts of D-biotin, 0.0002 parts of cholecalciferol, 0.00007 parts of cyanocobalamin, and 6.93763 parts of maltodextrin.

7. The nutritional composition according to claim 1, **characterized in that** it comprises the following raw materials in the pre-determined mass ratio:
257 parts of proteinaceous substances, 383 parts of fatty substances, 632 parts of carbohydrates, 60 parts of dietary fiber substances, 5.1 parts of complexed trace minerals, 106 parts of complexed major minerals, 12.9 parts of multi-vitamins, 6.21 parts of choline bitartrate, 1.1 parts of L-carnitine, and 0.42 parts of taurine;
the proteinaceous substance is composed of 47 parts of whey protein, 112 parts of soy protein, and 98 parts of casein;
the fatty substance is the microcapsulated vegetable oil powder made from 66 parts of medium-chain triglycerides, 79 parts of sunflower seed oil, 51 parts of soybean oil, 72 parts of low erucic acid rapeseed oil, and 115 parts of edible carriers; the edible carriers are composed of 68.66 parts of maltooligosaccharides, 31 parts of sodium caseinate, 4 parts of mono- and di-glycerides of fatty acid, 0.3 parts of vitamin E, 8.3 parts of sodium tripolyphosphate, 0.04 parts of ascorbyl palmitate, and 3 parts of sodium ascorbate;
the carbohydrate is composed of 420 parts of maltodextrin and 212 parts of solid corn syrup;
the dietary fiber substance is composed of 18 parts of galactooligosaccharides and 42 parts of fructooligosaccharides;
the complexed trace minerals are composed of 0.03 parts of copper sulfate, 0.81 parts of ferric pyrophosphate, 0.07 parts of manganese sulfate, 0.0023 parts of sodium selenite, 0.25 parts of zinc citrate, 0.0019 parts of potassium iodide, and 3.636 parts of maltodextrin;
the complexed major minerals are composed of 10.9 parts of tricalcium phosphate, 12.5 parts of calcium carbonate, 3.2 parts of magnesium carbonate, 17.6 parts of potassium chloride, 21.4 parts of potassium citrate, 32.6 parts of sodium citrate, and 7.8 parts of maltodextrin;
the multi-vitamins are composed of 3 parts of sodium L-ascorbate, 0.76 parts of vitamin E, 0.28 parts of nicotinamide, 0.12 parts of calcium D-pantothenate, 0.03 parts of pyridoxine hydrochloride, 0.05 parts of thiamine hydrochloride, 0.03 parts of vitamin A palmitate, 0.04 parts of riboflavin, 0.0042 parts of folic acid, 0.002 parts of phylloquinone, 0.0004 parts of D-biotin, 0.0001 parts of cholecalciferol, 0.00006 parts of cyanocobalamin, and 8.58324 parts of maltodextrin.

8. A method for preparing the nutritional composition according to any one of claims 1-7, **characterized in that** it comprises the following steps:
1) The raw materials are weighed according to the pre-determined ratio;
2) Choline bitartrate, L-carnitine, taurine, multi-vitamins, and complexed trace minerals are mixed, and stirred at 20-30 rpm for 10-20 min, to obtain premix 1; dietary fiber substances and whey protein are mixed, and stirred at 20-30 rpm for 10-20 min, to obtain premix 2; complexed major minerals and soy protein are mixed, and stirred at 20-30 rpm for 10-20 min, to obtain premix 3;
3) Said premix 1, premix 2, and premix 3 obtained in step 2) are mixed at 20-30 rpm for 20-30 min, to obtain total mix 1; solid corn syrup, casein, and total mix 1 are mixed at 20-30 rpm for 30-60 min, to obtain total mix 2; fatty substances, maltodextrin, and total mix 2 are mixed at 20-30 rpm for 40-60 min, to obtain the nutritional composition.

9. The use of the nutritional composition according to any one of claims 1 to 7 in the manufacture of food or medicaments for enhancing immunity, strengthening bones, and/or improving constipation, **characterized in that** the food or medicaments have the effects of enhancing immunity, increasing bone density, and/or promoting bowel movements.
